# EUROPEAN PATENT APPLICATION

(11) **EP 0 897 703 A2**
(43) Date of publication of application: **24.02.1999**
(21) Application number: 98306461.9
(22) Date of filing: 13.08.1998
(51) Int. Cl.: A61F 2/34, A61F 2/46, A61F 2/30

(54) **Cutting template**

(30) Priority: 14.08.1997 GB 9717294
(71) Applicant: Johnson & Johnson Medical Limited, Ascot, Berkshire SL5 9EY (GB)
(72) Inventor: Naybour, John, Flintshire, Wales (GB)
(74) Representative: Fisher, Adrian John

(57) **Abstract**

A cutting template (1) for use in trimming a flange of an acetabular prosthesis. The cutting template (1) has a cup portion (2) and a flange portion (4). The cup portion (2) is adapted to accommodate a cup portion of an acetabular prosthesis. The cutting template (1) sits within a patient's acetabulum and the flange portion (4) can be trimmed to fit precisely the acetabulum rim. Spacer elements in the form of projections (8) from the cup portion (2) of the cutting template (1) are provided to allow for the cement mantle thickness between the acetabular prosthesis and the patient's acetabulum.

## Description

This invention relates to a cutting template for use in trimming a flange of an acetabular prosthesis.

Acetabular prostheses can be provided with an integral flange, for example as described in UK patent no. GB1563334B. The flange extends from the acetabular cup prosthesis to fit closely within the patient's acetabulum. The flange increases the pressure on cement attaching the acetabular prosthesis to the acetabulum by preventing the egress of the cement from the acetabulum.

In order to be effective, a flange of an acetabular prosthesis must have a close fit within the patient's acetabulum. As each patient differs in shape, the flange of the acetabular prosthesis to be used must be trimmed to suit an individual patient.

The known prior art includes trimming aids of the form described in UK patent no. GB2086729B. This form of known trimming aid is formed of two components, a first component in the form of a cup which imitates the form of an acetabular cup prosthesis and is insertable in the patient's acetabulum. The second component is in the form of a trimmable flange which has a protrusion which fits into the socket of the cup component. The combined components are positioned in a patient's acetabulum and the flange of the second component can then be trimmed to fit precisely in the patient's acetabulum.

The second component of the trimming aid can then be placed in an acetabular prosthesis which includes an integral flange and the flange of the prosthesis can be cut in accordance with the shape of the flange of the trimming aid.

The use of a trimming aid ensures that the flange of the acetabular prosthesis fits precisely into the mouth of the cavity of the acetabulum. The trimming operation may need to be repeated several times in order to achieve a precise fit and the use of the trimming aid avoids unnecessary waste of the prosthesis by inaccurate trimming.

The known trimming aids have the disadvantage that they comprise two components which adds to the expense of the trimming aid.

According to the present invention, there is provided a cutting template for use in trimming a flange of an acetabular prosthesis the cutting template comprising a cup portion and a trimable flange portion, wherein the cup portion is adapted to accommodate a cup portion of an acetabular prosthesis.

Preferably, the cutting template includes spacer means disposed on the cup portion for defining a distance between the cup portion of the acetabular prosthesis and a patient's acetabulum.

Preferably, the spacer means is in the form of spacer elements which project a uniform distance from the cup portion of the cutting template.

Preferably, the spacer elements are in the form of projections on the inner or outer surface of the cup portion of the cutting template. The projections may be arcuate and may include an equatorial ring. The projections may be formed by indentations in the inner or outer surface of the cup portion of the cutting template.

Preferably, the flange portion extends outwardly from the open edge of the cup portion and is curved towards the outer surface of the cup portion.

Preferably, the flange portion has a lip on its edge.

Preferably, the cutting template is formed of a material which can be cut.

Preferably, the cutting template is formed of a transparent, plastics material.

Preferably, the cutting template can be vacuum formed as a single piece.

Embodiments of a cutting template in accordance with the present invention will now be described, by way of example only, with reference to the accompanying drawings in which:

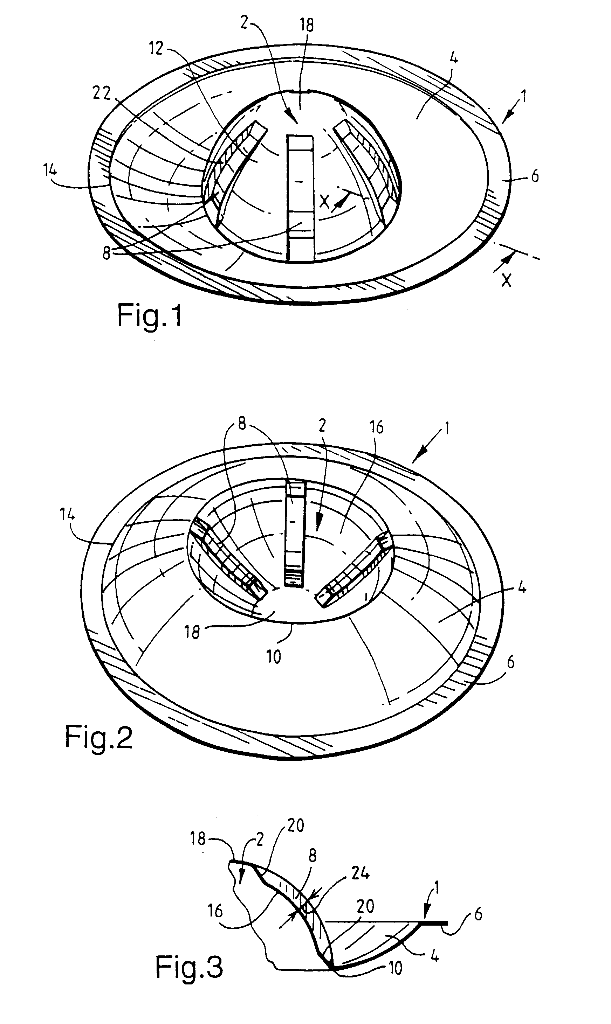

Figure 1 is a perspective view from above of a first embodiment of the cutting template;
Figure 2 is a perspective view from below of the cutting template of Figure 1;
Figure 3 is a section on line X-X of Figure 1 showing a cross-section of the cutting template;
Figure 4 is a perspective view from above of a second embodiment of the cutting template;
Figure 5 is a perspective view from below of the cutting template of Figure 4; and
Figure 6 is a section on line Y-Y of Figure 4 showing a cross-section of the cutting template.

Referring to the drawings, a first embodiment of a cutting template 1 for use in trimming a flange of an acetabular prosthesis is described.

The cutting template 1 is formed by vacuum as a single piece of transparent plastics material.

The cutting template 1 has a cup portion 2 of substantially hemisperical form and a flange portion 4. The flange portion 4 extends from the open edge 10 of the cup portion 2. The flange portion 4 is curved in a direction towards the outer surface 12 of the cup portion 2.

The edge 14 of the flange portion 4 has a lip 6 which extends a short distance outwardly from the edge 14 of the flange portion 4.

The cup portion 2 is provided with a plurality of spacer elements in the form of projections 8. The projections 8 project inwardly from the inner surface 16 of the cup portion 2. The projections 8 are arcuate extending from near the pole 18 of the cup portion 2 to the open edge 10 of the cup portion 2. In this embodiment, six projections 8 are provided.

The projections 8 have a uniform height 24 with tapered ends 20 and perpendicular sides 22. The projections 8 are formed by indentations in the outer surface 12 of the cup portion 2.

In use, the cutting template 1 is placed in the patient's acetabulum with the cup portion 2 positioned where the cup portion of the prosthesis is required. The flange portion 4 is marked where it needs to be cut. As the cutting template 1 is formed with transparent material, the acetabulum rim can be seen through the flange portion 4 of the cutting template 1 whilst the cutting template 1 is being cut.

Once the flange portion 4 of the cutting template 1 has been cut to fit precisely in the patient's acetabulum, it is removed from the acetabulum and the cup portion 2 cutting template 1 is placed over the cup portion of the acetabular prosthesis.

The projections 8 contact the outer surface of the cup portion of the acetabular prosthesis and space the inner surface 16 of the cup portion 2 of the cutting template 1 a uniform distance of height 18 from the outer surface of the cup portion of the acetabular prosthesis. The projections 8 have the purpose of allowing an additional uniform space for the cement mantle thickness between the cup portion of the acetabular prosthesis and the acetabulum.

The flange of the acetabular prosthesis can be trimmed precisely following the shape of the trimmed flange portion 4 of the cutting template 1.

The trimmed acetabular prosthesis can be positioned within the patient's acetabulum and a precise fit will be obtained with allowance for the cement mantle.

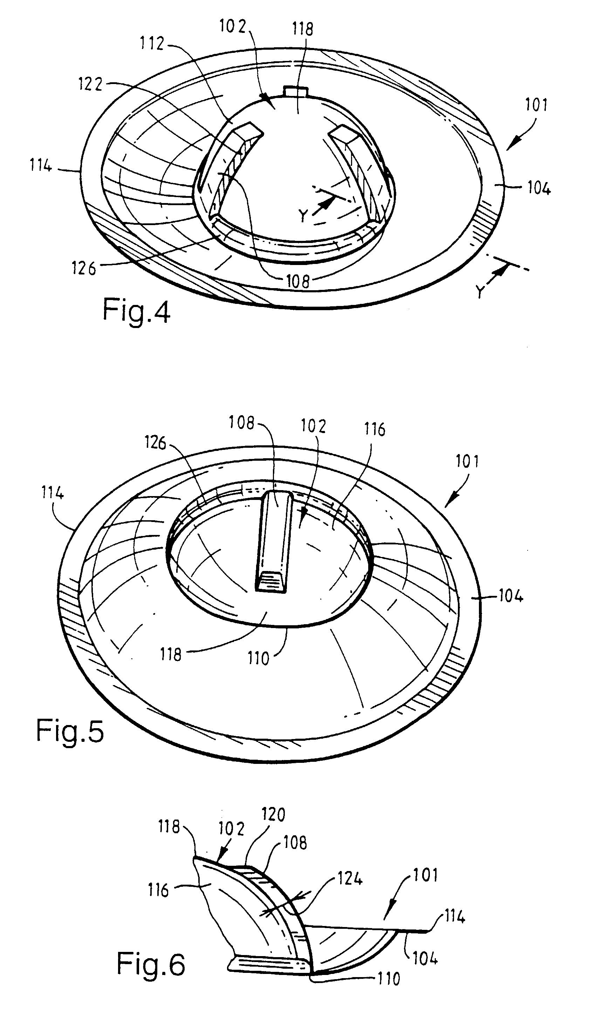

A second embodiment of a cutting template 101 for use in trimming a flange of an acetabular prosthesis is also described. Reference is made to Figures 4 to 6 which show the second embodiment of the cutting template 101.

The second embodiment of the cutting template 101 is similar to that of the first embodiment and has a cup portion 102 and a flange portion 104.

In the second embodiment, a plurality of spacer elements are provided in the form of projections 108 which project from the outer surface 112 of the cup portion 102. In the given example, there are three projections 108 in the form of arcuate indentations in the inner surface 116 of the cup portion 102.

In addition, a further spacer element is provided by a raised equatorial ring 126 adjacent the open edge 110 of the cup portion 102.

The arcuate projections 108 extend from an area close to the pole 118 of the cup portion 102 and are joined to the raised equatorial ring 126. The ends of the projections 108 adjacent the pole 118 are tapered 120. The projections 108 and the raised equatorial ring 126 have curved edges 122. The projections 108 and the raised equatorial ring 126 have a uniform height 124 from the outer surface 112 of the cup portion 102.

In the second embodiment, the edge 114 of the flange portion 104 does not have a lip portion.

In use, the cutting template 101 is used in the same way as the cutting template 1 of the first embodiment. The projections 108 and the raised equatorial ring 126 contact the patient's acetabulum in place of the outer surface 12 of the cup portion 2 of the first embodiment. Once the flange portion 104 has been trimmed, the cutting template 101 can be placed over the acetabular prosthesis with the inner surface 116 of the cup portion 102 contacting an outer surface of the acetabular prosthesis.

Modifications and improvements can be made to the foregoing without departing from the scope of the present invention.

## Claims

1. A cutting template (1,101) for use in trimming a flange of an acetabular prosthesis the cutting template (1,101) comprising a cup portion (2,102) and a trimable flange portion (4,104), wherein the cup portion (2,102) is adapted to accommodate a cup portion of an acetabular prosthesis.

2. A cutting template (1,101) as claimed in claim 1, wherein the cutting template (1,101) includes spacer means disposed on the cup portion (2,102) for defining a distance between the cup portion of the acetabular prosthesis and a patient's acetabulum.

3. A cutting template (1,101) as claimed in claim 2, wherein the spacer means is in the form of spacer elements which project a uniform distance from the cup portion (2,102) of the cutting template (1,101).

4. A cutting template (1,101) as claimed in claim 2 or claim 3, wherein the spacer elements are in the form of projections (8,108) on the inner (16,116) or outer surface (18,118) of the cup portion (2,102) of the cutting template (1,101).

5. A cutting template (1,101) as claimed in claim 4, wherein the projections (8,108) are arcuate.

6. A cutting template (1,101) as claimed in claim 4, wherein one of the projections (8,108) is an equatorial ring (126).

7. A cutting template (1,101) as claimed in any of claims 4 to 6, wherein the projections (8,108) are formed by indentations in the inner (16,116) or outer surface (1,118) of the cup portion (2,102) of the cutting template (1,101).

8. A cutting template (1,101) as claimed in any of the preceding claims, wherein the flange portion (4,104) extends outwardly from the open edge of the cup portion (2,102) and is curved towards the outer surface (18,118) of the cup portion (2,102).

9. A cutting template (1,101) as claimed in any of the preceding claims, wherein the flange portion (4,104) has a lip on its edge (14,114).

10. A cutting template (1,101) as claimed in any of the preceding claims, wherein the cutting template (1,101) is formed of a material which can be cut.

11. A cutting template (1,101) as claimed in any of the preceding claims, wherein the cutting template (1,101) is formed of a transparent, plastics material.

12. A cutting template (1,101) as claimed in any of the preceding claims, wherein the cutting template (1,101) can be vacuum formed as a single piece.
